# EUROPÄISCHE PATENTSCHRIFT

(11) **EP 0 975 385 B1**
(45) Veröffentlichungstag und Bekanntmachung des Hinweises auf die Patenterteilung: **10.12.2003**
(21) Anmeldenummer: 98922713.7
(22) Anmeldetag: 15.04.1998
(51) Int. Cl.: A61M 29/02

(54) **VORRICHTUNG ZUR GEBURTSVORBEREITUNG UND GEBURTSERLEICHTERUNG SOWIE VERFAHREN ZUR GYMNASTISCHEN GEBURTSVORBEREITUNG**
DELIVERY PREPARATION AND FACILITATION DEVICE AND PREPARATORY GYMNASTICS
DISPOSITIF DE PREPARATION ET DE FACILITATION DE L'ACCOUCHEMENT ET GYMNASTIQUE PREPARATOIRE

(30) Priorität: 15.04.1997 DE 19715724
(43) Veröffentlichungstag der Anmeldung: 02.02.2000
(73) Patentinhaber: Horkel, Wilhelm, 82319 Starnberg (DE)
(72) Erfinder: Horkel, Wilhelm, 82319 Starnberg (DE)
(74) Vertreter: Möhring, Friedrich
(86) Internationale Anmeldenummer: EP9802193
(87) Internationale Veröffentlichungsnummer: WO98046298

(56) Entgegenhaltungen:
- DE-C- 3 800 744
- DE-C- 3 803 727
- FR-A- 592 104
- US-A- 2 849 001
- US-A- 2 849 002
- US-A- 3 480 017
- US-A- 3 626 949
- US-A- 4 832 691

## Beschreibung

Die Erfindung betrifft eine Vorrichtung zur Geburtsvorbereitung und -erleichterung der schwangeren Frau, mit einem expandierabren länglichen Körper, der im Bereich des Gebärkanalausgangs derart positionierbar ist, daß er sich teilweise in der Scheide, teilweise außerhalb dieser befindet; sie betrifft ferner ein Verfahren zur gymnastischen Geburtsvorbereitung unter Verwendung einer derartigen Vorrichtung.

In der BRD gibt es ca. 850 000 Geburten im Jahr. Laut neuester verfügbarer Daten der Perinatalstatistik von 1995 beträgt die Häufigkeit der Dammschnitte (abzügl. der Sectiofrequenz von 17,4 %) bei Erstgebärenden und Mehrgebärenden insgesamt 58,9 %. Das bedeutet, daß jährlich in Deutschland ca. 500.650 Dammschnitte erfolgen (Kommission für Perinatologie und Neonatologie BPE-Jahresbericht 1995). Bei ca. 20% aller Geburten kommt noch ein Dammriss hinzu, wodurch die Morbidität (Risiko von Folgeschäden) zusätzlich erhöht wird. Trotz zahlreicher althergebrachter und moderner Methoden der Geburtsvorbereitung und der Geburtserleichterung (z.B. READ, LEBOYER, ODENT) bleiben die o.g. Zahlen seit Jahrzehnten in ihrer Relation zueinander nahezu unverändert. So vermochte selbst die neuzeitliche geburtsvorbereitende Schwangerschaftsgymnastik und Schwangerenberatung der Hebammen diese Zahlen nur unwesentlich zu verbessern.

Der Dammschnitt (Episiotomie), 1742 erstmals von OULD beschrieben hat auch nach 250 Jahren nichts von seinem Schrecken eingebüßt. Obwohl als "Entlastung" der Frau und zur Beschleunigung der Geburt gedacht und durchgeführt, wird er noch immer von vielen Frauen wegen seiner Schmerzhaftigkeit gefürchtet und wegen seiner schlimmen Folgen gehaßt. Die Vermeidung der Episiotomie ist daher in der Fachwelt ein lang gehegtes Bedürfnis, verbunden mit dem Ziel, den Gebärenden die Geburt zu erleichtern und angstfreier zu gestalten.

Ein zusätliches Problem der modernen Geburtshilfe besteht darin, daß sich das Mißverhältnis zwischen kindlichem Köpfchen und weiblichem Becken vergrößert. Der Kopfumfang der Neugeborenen nimmt stetig zu, so daß das von HAUSMANN erstellte Meßprogramm für die Normgrößen des Biparietalen Durchmessers (BIP) im Ultraschall immer wieder nach oben korrigiert werden muß. Die Ursache hierfür dürfte zum einen in der zunehmenden Licht-, Lärm- und Reizüberflutung (z.B. Neonlicht, Elektrosmog, Autolärm etc.) der Schwangeren zu sehen sein; zum anderen im heute naturgegebenen, modernen, androgynen Frauentyp (groß gewachsen, schmales weibliches Becken und schlanke Hüften), was naturgemäß die Geburt erschwert.

Zur Behandlung von Senkungs- und Inkontinenzbeschwerden bei Frauen ist es bekannt, einen aufblasbaren Ballon hoch in die Scheide einzuführen, um damit im aufgeblasenen Zustand einen mechanischen Verschluß der Harnblase mit Anhebung der Gebärmutter hervorzurufen. Beispielsweise ist in EP 0663197 Al ein formstabiler Stützkörper aus gummielastischem Material mit unterschiedlichen Wanddickenbereichen beschrieben, der sich von einem Basisbereich, mit dem ein Schlauchstück dichtend verbunden ist, in Richtung auf eine Deckfläche am anderen Ende des Stützkörpers hin konisch erweitert. Die Deckfläche ist ringförmig verstärkt und besitzt einen weichen, konkav ausgebildeten Innenbereich. Der Stützkörper wird mit der Basis nach unten weisend in die Scheide eingesetzt und zur Stützung der Scheidenwand stundenweise in situ belassen.

In US 3626949 Al ist ein Cervical Dilator beschrieben, der jedoch in der Praxis keine Bedeutung erlangt hat. Es handelt sich um eine PVC-Hülle mit Schlauchanschluß zum Einfüllen eines fluiden Mediums unter Druck. Die Hülle wird zusammengefaltet in den Cervixkanal eingeführt und dann periodisch unter Druck aufgeweitet, wobei sich die Hülle radial gegen die Cervixwand ausdehnt und dabei Wehen simuliert. Zwischen gegenüberliegenden Hüllenenden befindet sich eine in radialer Richtung nicht aufweitbare Taille, welche innerhalb des Randes der Cervix Uteri (Muttermund) positioniert ist und damit verhindert, daß die expandierte Taille unter Druck in axialer Richtung ausweicht.

Ein weiterer Cervical-Dilator ist in US 3480017 Al beschrieben. Dieser benutzt eine zu einem im wesentlichen scheibenförmigen Körper aufblasbare Hülle, die bei der Anwendung innerhalb des Muttermunds positioniert und mittels eines Füllrohrs, welches Öffnungen innerhalb der Hülle besitzt, aufgeblasen wird. Die Hülle weist eine umlaufende Einschnürung auf, derart, daß sich eine Rinne in der zylindrischen Außenwand der zur Scheibe aufgeblasenen Hülle bildet. Die in der Rinne abgestützte Öffnung des Muttermunds soll auf diese Weise zum Zwecke der Geburtseinleitung gedehnt werden können. Die Anwendung der bekannten Vorrichtung ist nur durch einen Arzt möglich, der darin geübt ist, die Hülle an dem durch das Füllrohr gebildeten Stiel vorsichtig an die Cervix heranzubewegen, ohne dabei die Fruchtblase oder das kindliche Köpfchen zu verletzen. Durch seine flache scheibenförmige Form ist der bekannte Cervical-Dilator speziell nur für diese Anwendung geeignet.

Ein in FR-592104-A beschriebener, in das Perineum einsetzbarer Dilatator besteht aus einem länglichen Ballon aus elastischem Material, der sich durch Aufblasen zwar lateral erweitern läßt, in seiner Längsausdehnung jedoch begrenzt ist. Durch methodische Anwendung und progressives Expandieren kann damit erreicht werden, daß das derart trainierte Gewebe bei der Geburt nachgibt, ohne zu reißen.

Dabei kann der Ballon über ein Dreiwegeventil auch intermittierend betätigt werden. Bei der Anwendung ist darauf zu achten, daß der Ballon nur so weit in die Vagina eingeführt wird, daß dessen Mittelabschnitt im Bereich des Perineums sitzt. Beim Aufpumpen soll der Ballon innerhalb des Perineums eine ringförmige Engstelle ausbilden, durch welche er in axialer Richtung fixiert wird.

In der Ausführungsform des Ballons mit einem zentralen Füllrohr besteht die Gefahr, daß dessen inneres Ende beim Einführen zu nahe an die Portio Uteri geschoben wird, wo ernste Verletzungen (Blutungen, Blasensprung) möglich sind.

Im Falle der Ausführungsform des Ballons mit zugfesten Innenbändern, die dessen Längsausdehnung verhindern, besteht die Gefahr, daß der Ballon beim Aufpumpen ab einem bestimmten Füllungsgrad plötzlich nach innen in Richtung Muttermund katapultiert wird, wo es zu den oben schon angesprochenen ernsten Verletzungen kommen kann.

Die bekannte Vorrichtung sollte daher nur unter ärztlicher Aufsicht eingesetzt werden, zumal es für die schwangere Frau wegen des entsprechenden Bauchumfangs praktisch unmöglich ist, die richtige axiale Lage des Ballons herauszufinden.

Demgegenüber besteht die Aufgabe der vorliegenden Erfindung darin, die eingangs genannte Vorrichtung derart auszugestalten, daß ihre Anwendung für die schwangere Frau angenehm und gefahrlos ist und von ihr selbst leicht durchgeführt werden kann; letztlich soll sie wesentlich dazu beitragen, daß mit der Geburt einhergehende Dammrisse bzw. Episiotomien weitgehend vermieden werden. Damit einhergehend soll einer unerwünschten Senkung des Beckenbodens, einer später eintretenden Harninkontinenz und Sexualstörungen durch postpartal auftretende organische und psychosomatische Komplikationen entgegengewirkt werden.

Diese Aufgabe wird durch eine Vorrichtung mit den Merkmalen des Anspruchs 1.

Die erfindungsgemäße Vorrichtung wird durch einen expandierbaren länglichen taillierten Körper gebildet, der im Bereich des Gebärkanalausgangs positionierbar ist, so daß er sich teilweise in der Scheide, teilweise außerhalb dieser befindet. Durch die Expansion des länglichen Körpers kann der Scheidenkanal vorübergehend erweitert und der Geburtskanal sowie der Hymenalsaum zur Erleichterung der Geburt vorgedehnt werden. Mit diesem Trainings- und Dehnungseffekt können Episiotomien und Dammrisse sowie eine unerwünschte Senkung des Beckenbodens und damit verbundene Folgeerscheinungen weitgehend vermieden werden. Der expandierbare Körper hält dabei Distanz zur Cervix Uteri, die er zur Vermeidung von Irritationen keinesfalls tangieren darf.

Dadurch, daß der expandierbare längliche Körper tailliert ist, wobei die Taille seiner Positionieren im Bereich des Hymenalsaums dient, ist die Vorrichtung im Bereich des Hymenalsaums selbstzentrierend und selbstfixierend. Dies kann beispielsweise durch eine Gestaltung des Körpers als in seiner Längsachse liegende "8" erreicht werden. Dabei ist wesentlich, daß der expandierbare Körper in seiner Gesamtheit, also auch mit seiner Taille dehnbar ist, weil gerade dadurch gezielt die Erweiterung im Bereich des Geburtskanalausgangs trainierbar ist.

Nach einer vorteilhaften Weiterbildung ist vorgesehen, daß der Körper mittels einer variablen Fluidbefüllung expandierbar ist. Als Fluid können beispielsweise gelartige oder wäßrige Flüssigkeiten, die ggf. temperiert sind, oder Gase wie Luft Verwendung finden. In besonderen Fällen kann auch Atemluft, die mit den Mund eingeblasen wird, zum Einsatz kommen.

Weiterhin ist es vorteilhaft, wenn der Körper als Ballon ausgebildet ist, an dessen bei der Anwendung nach außen gerichtetem Ende ein Füllschlauch angeschlossen ist. Als hautfreundliches Material kommt dabei beispielsweise Latex oder ein Siliconprodukt zur Anwendung.

Nach einer bevorzugten Ausführungsform ist der Ballon aus zwei Halbschalen zusammengesetzt, die längs eines Umfangssaums miteinander verbunden sind. Der Umfangssaum ist dabei vorteilhafterweise im Bereich der Taille angeordnet. Dabei kann die außerhalb der Scheide positionierte Hälfte eine größere Wandstärke als die innenliegende Hälfte besitzen. Damit ist gewährleistet, daß sich der Ballon bei Druckerhöhung von selbst stabilisiert und schon ein geringer Druckanstieg zur vaginalen Dilatation führt.

Eine weitere vorteilhafte Ausführungsform sieht vor, daß die Vorrichtung eine am Füllschlauch des Ballons angeschlossene Luftpumpe mit Manometer aufweist. Durch die beispielsweise als Handpumpe ausgeführte Pumpe kann der expandierbare Körper schrittweise bis zu einem Maximaldruck, der bei ca. 160 mm Hg liegt und am Manometer ablesbar ist, aufgepumpt werden.

Am Manometer ist zweckmäßig eine Luftablaßschraube vorgesehen, die nach Beendigung der Therapie oder zur Entlastung während der Anwendung zum Ablassen des Druckes dient.

Zu beachten ist schließlich, daß die maximale Ausdehnung des Ballons quer zur Längsachse 9 bis 10 cm, vorzugsweise 9,4 bis 9,6 cm beträgt. Dies entspricht etwa dem durchschnittlichen Durchmesser eines kindlichen Köpfchens mit einem Kopfumfang von ca. 35 cm bei der Geburt.

Eine weitere Ausgestaltung sieht vor, daß am Manometer ein Sicherheitsventil angeordnet ist, um den maximalen Druck zu begrenzen. Der Druck wird dabei beispielsweise auf 200 mm Hg begrenzt. Bei Überschreitung des Grenzdruckes öffnet sich das Sicherheitsventil.

Nach einer anderen vorteilhaften Ausführungsform ist der aus zwei Halbschalen zusammengesetzte Ballon derart aufgebaut, daß die Halbschalen zwei gasdicht voneinander getrennte Kammern bilden. Hierdurch können die Kammern jeweils unabhängig voneinander befüllt bzw. entlüftet werden, wodurch sich der Ballon individuell an die Bedürfnisse der Patientin anpassen läßt. Dabei ist der zum Befüllen bzw. Entlüften der innerhalb der Scheide positionierten Halbschale vorgesehene Schlauch durch die andere Halbschale hindurch geführt. Beide Schläuche treten dann am nach außen gerichteten Ende des Ballons aus. Diese Vorrichtung ist von Vorteil vor allem bei Patientinnen, die bereits entbunden haben und bei denen der in den vorangegangenen Geburten eingetretene Dammriß zu schweren und schmerzhaften Vernarbungen geführt hat. Es ist zweckmäßig, diese Vorrichtung abwechselnd in Druckstufen von 10 mm Hg aufzupumpen, um eine wechselseitige Wirkung auf den Scheidenausgang auszuüben.

Eine vorteilhafte Weiterbildung dieser Ausführungsform ist, daß die in der Vagina liegende Halbschale mittels eines separaten Füllschlauchs befüllbar ist. Dabei kann der separate Füllschlauch entweder außerhalb der Vorrichtung verlaufen oder aber durch das Innere der außerhalb der Vagina liegenden Halbschale verlaufen, so daß beide Schläuche außerhalb des Gebärkanals nach außen gelangen. Weiterhin ist eine Variante vorteilhaft, bei der beide Schläuche konzentrisch ineinander verlaufen.

Schließlich ist es vorteilhaft, daß die Vorrichtung ein Dreiwege-Ventil zur Wahl des Befüllungsmodus der beiden Halbschalen aufweist. Dabei können die beiden getrennt voneinander befüllbaren Halbschalen jeweils einzeln bzw. gleichzeitig befüllt werden. Der Fülldruck ist jeweils für den entsprechenden Befüllmodus am Manometer ablesbar. Auch die Entlüftungsreihenfolge kann mittels des Dreiwege-Ventils gewählt werden. Bei konzentrisch verlaufenden Schläuchen kann ein entsprechend konstruktiv aufgebautes Dreiwege-Ventil vorgesehen werden.

Neben der oben beschriebenen Vorrichtung betrifft die Erfindung noch ein Verfahren zur gymnastischen Geburtsvorbereitung, wobei die erfindungsgemäße Vorrichtung in der Weise zur Anwendung kommt, daß der expandierbare Körper teils außerhalb, teils innerhalb des Hymenalsaums positioniert wird, danach bis zu einer gewünschten Ausdehnung expandiert wird und schließlich im expandierten Zustand eine gewisse Zeit in seiner Position verbleibt. Vorteilhafterweise wird die Ausdehnung der Vorrichtung bei wiederholter Anwendung jedesmal vergrößert, um eine zunehmende Vordehnung des Geburtskanals zu erreichen. Das als Dehnübung durchführbare verfahren sollte ab der 36sten Schwangerschaftswoche mehrmals täglich durchgeführt werden. Zusätzlich ist es vorteilhaft, wenn der expandierte Körper nach einer gewissen Verweilzeit sachte in Richtung des Scheidenausgangs gezogen wird. Die Verweilzeit der Vorrichtung in der Scheide beträgt jeweils 10 - 20, vorzugsweise 15 Minuten je nach subjektivem Empfinden der Patientin: zu starke Dehnungsspannung signalisiert zu hohen Druck im Ballon. Abhilfe leistet hier die schrittweise Betätigung der Luftablaßschraube.

Die zahlreichen Vorteile des genannten Verfahrens sind leichtere Geburt durch bereits erreichte Vordehnung des Geburtskanals und des äußeren Scheidenringes (Hymenalsaum), schnellerer Geburtsvorgang, vor allem bei Erstgebärenden, bessere Sauerstoffversorgung des Kindes durch geringere Druckbelastung des kindlichen Köpfchens in der Austreibungsphase, Einsparung von Wehenmitteln, insbesondere bei Erstgebärenden Verringerung des Geburtsschmerzes und geringere Verkrampfung durch dehnbarere Muskulatur, deutliche Reduzierung von Narkosemitteln und Lokalanästhetika, weniger Risiko des allergischen Schocks auf lokale Betäubungsmittel, Verminderung der Periduralanästhesien und Epiduralanästhesien (Rückenmarksnarkose), weitgehender Verzicht auf den für die Gebärende immer schmerzhaften und damit gefürchteten Dammschnitt mit der unausweichlich folgenden Dammnaht, sowie schnellere Rückbildung der Scheidenmuskulatur durch Schonung des Musculus Bulbocavernosus, des Musculus Bulbospongiosus und des Musculus Sphincter Ani, da die Inzidenz von Macro- und Micro-Muskelrissen verringert wird.

Weitere Vorteile in der Folge einer problemfreien Geburt sind bessere Versorgung des Kindes durch die Mutter, die mangels Dammschnitt aktiver und agiler ist, also das Kind selbst versorgen kann und sich nicht krank und verletzt fühlt. Durch die erfolgte Schmerzfreiheit kommt es zu einer erhöhten Prolactin-Produktion, wodurch die Lactation (Stillen) begünstigt wird. Wichtig ist ferner, daß Physiologie, Funktion und Ästhetik von Scheide und Vulva im hohem Maße erhalten bleiben, und damit das spätere Sexualleben nicht beeinträchtigt wird. Eine kürzere Liegedauer in der

Klinik und die Möglichkeit einer ambulanten Geburt führen zur Entlastung der Krankenkassen.

Die Erfindung wird im folgenden anhand von Ausführungsbeispielen zusammen mit den beigefügten Zeichnungen beschrieben. Dabei zeigen:
- Fig. 1: eine Ausführungsform der erfindungsgemäßen Vorrichtung bei der Anwendung im entspannten Zustand;
- Fig. 2: die Ausführungsform der erfindungsgemäßen Vorrichtung nach Fig. 1, bei der Anwendung im expandierten Zustand;
- Fig. 3: einen Schnitt durch die Ausführungsform der erfindungsgemäßen Vorrichtung nach Fig. 1 und 2;
- Fig. 4: einen Schnitt durch eine weitere Ausführungsform einer Vorrichtung gemäß vorliegender Erfindung.

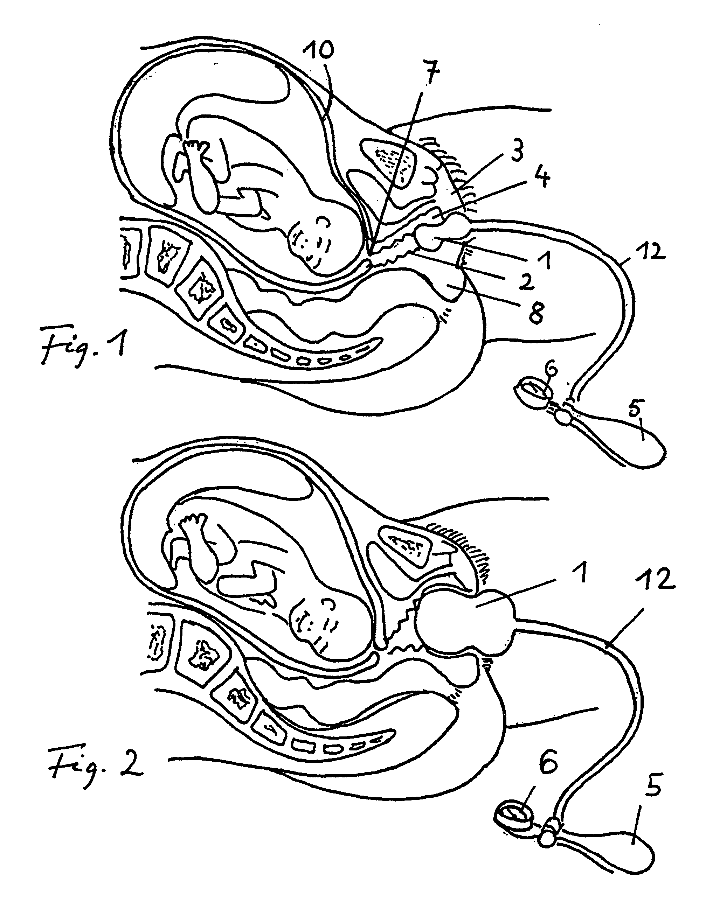

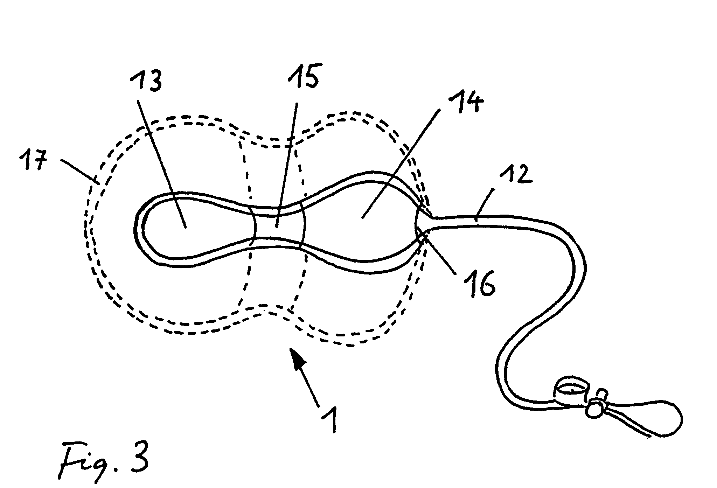

Fig. 1 - 3 zeigen den Ballon 1 zur pneumatischen Dilatation des Geburtskanals, welcher sich ausgehend vom Muttermund 7 des Uterus 10 in die Scheide 2 fortsetzt. Der oval und 8-förmig gestaltete expandierbare Ballon 1 mit medianer Taille 15 besitzt in seiner außerhalb der Scheide 2 liegenden Hälfte 14 eine größere Wandstärke als die innenliegende Hälfte 13. Die Taille 15 des Ballons 1 selbst ist verstärkt. Der Ballon 1 wird in nichtaufgeblasenem Zustand gefaltet, mit Gleitgel versehen und in die Scheide 2 eingeführt, so daß ca. 3 - 4 cm des Ballons 1 noch vor der Vulva 3 zu sehen sind. Dies ist wichtig, da besonders der äußere Bereich der Scheide 2, vor allem aber der Hymenalsaum 4 erweitert werden soll. Dies ist der empfindlichste und wegen der Gefahr des Dammrisses zugleich auch gefährdetste Teil des weiblichen Geburtskanals. Nun wird der Ballon 1 vorsichtig mit der Handpumpe 5 schrittweise in Stufen von 20 mm Hg bis zu einem Druck von max. 160 mm Hg, ablesbar am Manometer 6 aufgepumpt. Das von der Schwangeren subjektiv empfundene Dehnungsgefühl setzt hierbei die Grenze. Nach ca. 15 - 20 Min. wird die Vorrichtung 1 vorsichtig und sanft Richtung äußere Scheide gezogen, so daß die Patientin ein "Gebärgefühl" verspürt und dieses in den darauffolgenden Übungen vertieft, was zu einer erheblichen Anxiolyse führt. Diese Übung wird mehrmals täglich durchgeführt wobei die Patientin den Scheidenausgang kontinuierlich und völlig schmerzfrei erweitert. Wichtig ist, den gewünschten Durchmesser des Ballons durch entsprechendes Aufpumpen (Anzahl der Pumpstöße) außerhalb der Scheide zu messen. Der Druck am Manometer sollte 200 mm Hg nie übersteigen. Entsprechende Markierungen sind auf der Skala angebracht.

Der maximale Durchmesser der Taille soll max. 9,5 cm betragen, da der biparietale Durchmesser des kindlichen Köpfchens am Geburtstermin durchschnittlich diese Größe aufweist, entsprechend einem Kopfumfang von 35 cm. Wenn sich bei der Ultraschalluntersuchung durch den Arzt herausstellt daß es ein besonders großes Kind wird, so kann der Durchmesser der Dehnung ohne weiteres angepaßt werden. Doch sollte auch hier die Patientin die Größe des Ballons immer ihrem subjektiven Empfinden anpassen.

Als Bemessungsgrundlage für die erforderlichen Drücke bei der Dilatation des Geburtskanals dienen folgende Untersuchungsergebnisse: Nach wissenschaftlichen Arbeiten von R. CALDEIRO-BARCIA kann der intraamniale Druck bei normaler Wehentätigkeit auf bis zu 100 mm Hg ansteigen. Der systolische Blutdruck der Gebärenden (normalerweise 120 mm Hg) kann während der Presswehen in der Austreibungsperiode kurzfristig auf 180 bis über 200 mm Hg ansteigen. Der Druck der Uterusmuskulatur erreicht gegen Ende der Eröffnungsperiode einen Druck von bis zu 200 mm Hg gegen das umliegende Scheidengewebe. Nach C. LINDGREN erreicht der maximale Druck auf das kindliche Köpfchen im Bereich des Dammes 8 kurz vor der Geburt während der Presswehen Spitzenwerte von bis zu 300 mm Hg. Um bei der hier beschriebenen künstlichen Dilatation im physiologischen Bereich zu bleiben, wird eine Begrenzung auf 200 mm Hg empfohlen. Grundsätzlich sollte die Dilatation nicht vor der 36. Schwangerschaftswoche erfolgen.

Die Vorführung und Erklärung des Verfahrens zur Dilatation des Geburtskanals soll grundsätzlich an der Patientin durch den behandelnden Arzt oder durch die Hebamme erfolgen. Es wird empfohlen, die erste Dilatation unter laufender CTG-Kontrolle (Cardiotocographie = kontinuierliche Aufzeichnung von kindlichen Herztönen und Kontraktionen) durch den betreuenden Arzt oder die Hebamme durchführen zu lassen.

Die Reinigung nach Gebrauch erfolgt mit Seife und Wasser und Trocknen an der Luft. Eine Desinfektion mit handelsüblichen Desinfektionsmitteln wird empfohlen.

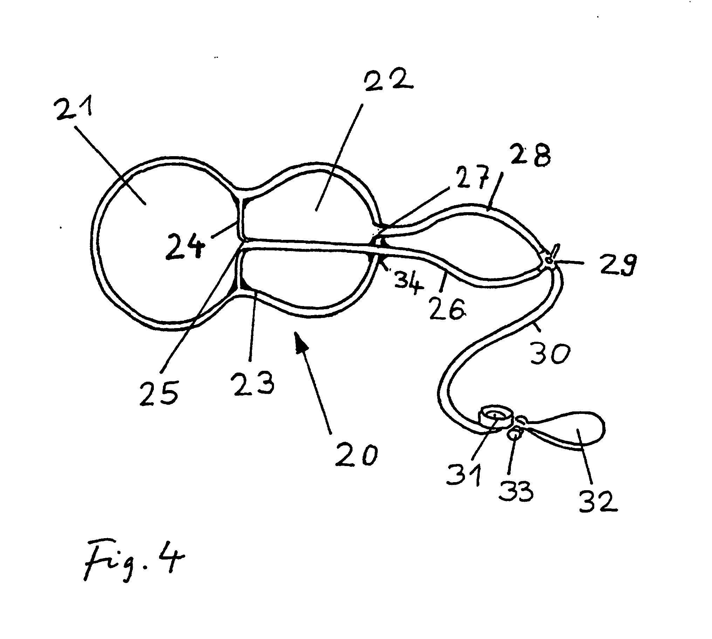

In Fig. 3 sind des weiteren der Schlauchanschluß 16 sowie der expandierbare Ballon 1 in seiner expandierten Stellung 17 angedeutet. Die beiden Halbschalen 13, 14 sind längs des Umfangssaums, der mit der Taille 15 zusammenfällt, miteinander überlappend verklebt bzw. verschweißt. Der Überlappungsbereich ist dabei gestrichelt angedeutet.

Fig. 4 zeigt einen Schnitt durch eine weitere Ausführungsform der Vorrichtung gemäß vorliegender Erfindung. Darin sind zwei getrennt voneinander aufblasbare Halbschalen 21, 22 des Ballons 20 dargestellt. Im Bereich der Taille 23 ist im Inneren des Ballons eine Membran 24 angeordnet, die eine Öffnung 25 aufweist, welche mit einem ersten Abzweigschlauch 26 verbunden ist. Die äußere Halbschale 22 ist über eine Wandöffnung 27 mit einem weiteren Abzweigschlauch 28 verbunden. Die beiden Abzweigschläuche 26, 28 sind mit einem Dreiwege-Ventil 29 verbunden, das wiederum über einen Schlauch 30 mit einem Manometer 31 und einer Handpumpe 32 verbunden ist. Schließlich ist noch eine Luftablaßschraube 33 zwischen Manometer 31 und Handpumpe 32 sowie eine elastische Schweißnaht 34 am Durchtritt des ersten Abzweigschlauches 26 durch die Wand der äußeren Halbschale 22 dargestellt.

Bei der Ausführungsform gemäß Fig. 4 können über das Dreiwege-Ventil 29 drei Stellungen eingestellt werden. In einer ersten Stellung ist die Verbindung zwischen dem Schlauch 30 und den beiden Abzweigschläuchen 26, 28 hergestellt. Hierdurch werden beide Kammern, d.h. die beiden Halbschalen 21, 22 gleichzeitig befüllt bzw. entlüftet. In einer weiteren Stellung wird eine Verbindung zwischen dem Schlauch 30 und dem Abzweigschlauch 26 zur inneren Halbschale 21 hergestellt, wobei die Verbindung zum zweiten Abzweigschlauch 28 unterbrochen ist. In einer dritten Stellung ist eine Verbindung zwischen dem Schlauch 30 und dem Abzweigschlauch 28 zur äußeren Halbschale 22 hergestellt, wobei die Verbindung zum anderen Abzweigschlauch 26 unterbrochen ist. Hierdurch kann die äußere Halbschale 22 durch die Handpumpe 32 befüllt bzw. mittels der Luftablaßschraube 33 entlüftet werden. Der Abzweigschlauch 26 zur inneren Halbschale 21 verläuft dabei durch die äußere Halbschale 22 direkt zu der Membran 24, an deren Öffnung 25 er mündet. Durch einen derartigen Zweikammeraufbau läßt sich das Volumen des Ballons 20 individuell an die Bedürfnisse der Benutzerin anpassen. Der Druck der jeweils zugeschalteten Ballonkammern läßt sich am Manometer 31 getrennt ablesen.

## Patentansprüche

1. Vorrichtung zur Geburtsvorbereitung und Geburtserleichterung der schwangeren Frau, mit einem expandierbaren länglichen Körper, der im Bereich des Gebärkanalausgangs derart positionierbar ist, daß er sich teilweise in der Scheide, teilweise außerhalb dieser befindet,
**dadurch gekennzeichnet,**
**daß** der Körper in seiner Gesamtheit expandierbar ausgebildet ist, wobei er eine mitexpandierbare Taille (15; 23) besitzt, die im Bereich des Hymenalsaums (4) selbstzentrierend positionierbar ist.

2. Vorrichtung nach Anspruch 1,
**dadurch gekennzeichnet,**
**daß** der Körper mittels einer variablen Luft- oder Fluidfüllung expandierbar ist.

3. Vorrichtung nach Anspruch 2,
**dadurch gekennzeichnet,**
**daß** der Körper als Ballon (1; 20) ausgebildet ist, an dessen bei der Anwendung nach außen gerichtetem Ende zumindest ein Füllschlauch (12; 28) angeschlossen ist.

4. Vorrichtung nach Anspruch 3,
**dadurch gekennzeichnet,**
**daß** der Ballon (1; 20) aus zwei Halbschalen (13, 14; 21, 22) zusammengesetzt ist, die längs eines Umfangssaums (15; 23) miteinander verbunden sind.

5. Vorrichtung nach Anspruch 4,
**dadurch gekennzeichnet,**
**daß** der Umfangssaum im Bereich der Taille (15; 23) angeordnet ist.

6. Vorrichtung nach Anspruch 4,
**dadurch gekennzeichnet,**
**daß** die Wandstärke der bei der Anwendung außerhalb der Scheide angeordneten Halbschale größer ist als die der anderen Halbschale.

7. Vorrichtung nach Anspruch 3,
**dadurch gekennzeichnet,**
**daß** sie eine am Füllschlauch (12; 30) des Ballons angeschlossene Luftpumpe (5; 32) mit Manometer (6; 31) umfaßt.

8. Vorrichtung nach Anspruch 7,
**dadurch gekennzeichnet,**
**daß** zwischen Luftpumpe (5; 32) und Manometer (6; 31) eine Luftablaßschraube (33) angebracht ist, mittels welcher der Druck im Ballon (1; 20) einstellbar ist.

9. Vorrichtung nach Anspruch 7,
**dadurch gekennzeichnet,**
**daß** ein Sicherheitsventil vorgesehen ist, um den maximalen Druck zu begrenzen.

10. Vorrichtung nach Anspruch 4,
**dadurch gekennzeichnet,**
**daß** die Halbschalen (21, 22) zwei gasdicht voneinander getrennte Kammern bilden.

11. Vorrichtung nach Anspruch 10,
**dadurch gekennzeichnet,**
**daß** die in die Vagina einsetzbare Halbschale (21) mittels eines separaten Füllschlauchs (26) befüllbar ist.

12. Vorrichtung nach Anspruch 10,
**dadurch gekennzeichnet,**
**daß** die Vorrichtung ein Dreiwege-Ventil (29) zur Wahl des Befüllungsmodus der beiden Halbschalen (21, 22) aufweist.

## Claims

1. Device for assisting and preparing the pregnant mother for the delivery, comprising an elongated, expandable element, which can be positioned in the region of the opening of the birth canal in such a manner that it is partially in the vagina and partially outside the vagina, **characterised in that** the element is designed to be expandable in its entirety, providing a coexpandable waist (15; 23), which can be positioned in a self-centring manner in the region of the edge of the hymen (4).

2. Device according to claim 1, **characterised in that** the element can be expanded by means of a variable air and/or fluid filling.

3. Device according to claim 2, **characterised in that** the element is designed as a balloon (1; 20), wherein at least one filling tube (12; 28) is connected at the end of the balloon which is directed outwards during use.

4. Device according to claim 3, **characterised in that** the balloon (1; 20) is composed of two half shells (13, 14; 21, 22), which are connected to one another along a circumferential seam (15; 23).

5. Device according to claim 4, **characterised in that** the circumferential seam is arranged in the region of the waist (15; 23).

6. Device according to claim 4, **characterised in that** the wall thickness of the half shell arranged outside the vagina during use is greater than that of the other half shell.

7. Device according to claim 3, **characterised in that** it provides an air pump (5; 32) with a manometer (6; 31) connected to the filling tube (12; 30) of the balloon.

8. Device according to claim 7, **characterised in that**, an air-release screw (33) is attached between the air pump (5; 32) and the manometer (6; 31), by means of which the pressure in the balloon (1; 20) can be adjusted.

9. Device according to claim 7, **characterised in that** a safety valve is provided, in order to limit the maximum pressure.

10. Device according to claim 4, **characterised in that**, the half shells (21, 22) form two separate chambers separated from one another in a gas-tight manner.

11. Device according to claim 10, **characterised in that** the half shell (21) for insertion into the vagina can be filled by means of a separate filling tube (26).

12. Device according to claim 10, **characterised in that** the device provides a three-way valve (29) for selecting the filling mode for the two half shells (21, 22).

## Revendications

1. Dispositif pour la préparation et la facilitation de l'accouchement de la femme enceinte, comprenant un corps allongé expansible qui peut être positionné dans la région de la sortie du canal utérin, de manière qu'il se trouve pour partie dans le vagin et pour partie à l'extérieur de celui-ci, **caractérisé en ce que** le corps, dans sa totalité, est expansible, et comporte un étranglement (15 ; 23) qui peut être positionné et se centre automatiquement dans la région de la vulve.

2. Dispositif selon la revendication 1, **caractérisé en ce que** le corps peut être expansé grâce à un remplissage variable d'air ou de liquide.

3. Dispositif selon la revendication 2, **caractérisé en ce que** le corps se présente sous la forme d'un ballon (1 ; 20), dont l'extrémité tournée vers l'extérieur en utilisation est connectée à au moins un tuyau souple de remplissage (12 ; 28).

4. Dispositif selon la revendication 3, **caractérisé en ce que** le ballon (1 ; 20) est formé de deux demi-coques (13, 14 ; 21, 22), qui sont assemblées l'une avec l'autre au niveau d'un bord périphérique (15 ; 23).

5. Dispositif selon la revendication 4, **caractérisé en ce que** le bord périphérique est disposé dans la région de l'étranglement (15 ; 23).

6. Dispositif selon la revendication 4, **caractérisé en ce que** l'épaisseur de paroi de la demi-coque qui en utilisation se trouve à l'extérieur du vagin, est supérieure à celle de l'autre demi-coque.

7. Dispositif selon la revendication 3, **caractérisé en ce qu'**il comprend une pompe à air (5 ; 32) avec manomètre (6 ; 31) connectée au tuyau souple de remplissage (12 ; 28) du ballon.

8. Dispositif selon la revendication 7, **caractérisé en ce qu'**entre la pompe à air (5 ; 32) et le manomètre (6 ; 31) est disposée une vis de purge d'air (33), à l'aide de laquelle la pression à l'intérieur du ballon peut être réglée.

9. Dispositif selon la revendication 7, **caractérisé en ce qu'**il est prévu une soupape de sûreté, aux fins de limiter la pression maximale.

10. Dispositif selon la revendication 7, **caractérisé en ce que** les demi-coques (21, 22) forment deux chambres distinctes, étanches au gaz.

11. Dispositif selon la revendication 10, **caractérisé en ce que** la demi-coque (21) à introduire dans le vagin peut être remplie à l'aide d'un tuyau souple de remplissage (26) séparé.

12. Dispositif selon la revendication 10, **caractérisé en ce que** le dispositif comporte un distributeur trois voies (29) pour le choix du mode de remplissage des deux demi-coques (21, 22).
